Europäisches Patentamt

European Patent Office

Office européen des brevets

(19)

(11) Numéro de publication : **0 122 833**
**B1**

(12) **FASCICULE DE BREVET EUROPÉEN**

(45) Date de publication du fascicule du brevet :
**03.12.86**

(51) Int. Cl.⁴ : **G 01 N 33/543**

(21) Numéro de dépôt : **84400531.4**

(22) Date de dépôt : **14.03.84**

(54) **Procédé de dosage en phase hétérogène de substances biologiques antigéniques, mettant en oeuvre des conjugués hybrides d'anticorps et réactifs pour sa mise en oeuvre.**

(30) Priorité : **15.03.83 FR 8304251**

(43) Date de publication de la demande :
**24.10.84 Bulletin 84/43**

(45) Mention de la délivrance du brevet :
**03.12.86 Bulletin 86/49**

(84) Etats contractants désignés :
**AT BE CH DE FR GB IT LI LU NL SE**

(56) Documents cités :
**EP-A- 0 014 530**
**EP-A- 0 034 050**
**EP-A- 0 041 426**
**EP-A- 0 074 271**
**FR-A- 2 415 301**
**GB-A- 2 001 172**
**US-A- 4 185 084**

(73) Titulaire : **INSTITUT PASTEUR**
**28, rue du Docteur Roux**
**F-75715 Paris Cedex 15 (FR)**

(72) Inventeur : **Guesdon, Jean-Luc**
**12, rue du Hameau**
**F-75015 Paris (FR)**
Inventeur : **Avrameas, Eustrate**
**1 Rue Sarasate**
**F-75015 Paris (FR)**

(74) Mandataire : **Phélip, Bruno et al**
**c/o Cabinet Harlé & Phélip 21, rue de la Rochefoucauld**
**F-75009 Paris.(FR)**

EP 0 122 833 B1

## Description

La présente invention concerne un procédé de dosage en phase hétérogène de substances biologiques antigéniques mettant en œuvre des dérivés hybrides d'anticorps, tels que des conjugés préparés par couplage d'anticorps antiimmunoglobulines et d'anticorps anti-enzymes et les réactifs pour sa réalisation.

On connaît des procédés de dosages immunochimiques, dans lesquels on fixe sur une matrice un anticorps dirigé contre la substance à doser, et on utilise une enzyme comme marqueur dosable par les techniques classiques, ladite enzyme étant liée de manière covalente à un anticorps dirigé contre la substance à doser. Ce procédé est décrit par exemple par S. AVRAMEAS, J. L. GUESDON dans « Solid phase enzyme immuno-assays » publié dans « Applied Biochemistry and Bio-engineering Academic Press Editeur, 1981, 73, 471-482 ». Toutefois, la préparation du produit résultant du couplage de l'anticorps et de l'enzyme est délicate et l'enzyme est fréquemment dénaturée, partiellement, au cours de cette réaction.

On connaît aussi un procédé de dosage en phase hétérogène dans lequel on fixe la substance à doser sur un excès d'antigène immobilisé pour faire ensuite réagir un produit de couplage lectine/anti-gène immobilisé ou lectine/substance à doser avant de révéler la lectine fixée par un composé porteur de fractions glycosidiques ; un tel procédé est décrit dans la demande de brevet européen -A-0 041 426.

On a maintenant trouvé un nouveau procédé de dosage de substances biologiques antigéniques, en phase hétérogène, et avec marqueur enzymatique mettant en œuvre un nouveau produit intermédiaire coupleur qui est le résultat du couplage covalent de deux anticorps, l'un dirigé contre la substance à doser, l'autre dirigé contre une enzyme qui est choisie comme le marqueur du système et sera révélée par les techniques classiques. L'invention concerne aussi ce nouveau produit et le système de réactifs nécessaire à la mise en œuvre du procédé.

Le procédé de dosage de substances biologiques antigéniques selon l'invention consiste à 1) mettre le milieu contenant la substance à doser en contact avec un anticorps de ladite substance fixé sur une matrice solide, insoluble dans le milieu biologique contenant la substance, séparer la matrice modifiée sur laquelle sont fixés l'anticorps et la substance à doser ; 2) faire réagir ladite matrice modifiée avec un produit coupleur intermédiaire ayant simultanément une affinité pour la substance à doser et pour une enzyme, ledit produit résultant de la fixation covalente de deux anticorps dont l'un est dirigé contre la substance à doser et l'autre contre l'enzyme, puis séparer la matrice ayant réagi ; 3) mettre l'enzyme isolée ou non au contact de la matrice ainsi traitée au cours d'une étape dite de marquage, puis séparer la matrice ainsi traitée ; 4) doser l'enzyme fixée, au cours d'une étape dite de révélation en mesurant l'activité enzymatique de la matrice traitée mise en présence d'une solution du substrat de l'enzyme. Ce procédé est également applicable au dosage des anticorps. Dans ce cas, l'antigène est fixé sur la matrice.

Lors de son dosage, l'enzyme sera donc fixée à la matrice par l'intermédiaire d'un complexe « anticorps-antigène », lui-même lié de manière non covalente au produit intermédiaire composé d'un anticorps lié à un autre anticorps par l'intermédiaire d'un pont fixé par liaisons covalentes.

Une variante du procédé de dosage selon l'invention consiste, lors de la deuxième étape, à faire réagir la matrice modifiée avec un mélange préparé préalablement du produit intermédiaire de couplage et de l'enzyme choisie.

Le procédé selon l'invention est approprié pour doser toute substance biologique pour laquelle on peut préparer des anticorps et notamment des antigènes, des anticorps, des haptènes, des immunoglobulines, des protéines, des hormones. Les enzymes utilisables comme marqueurs, sont d'origine végétale ou bactérienne ou animale et purifiées ou non, et solubles ou fixées sur des particules telles que des particules de diamètre compris entre 0,5 à 20 $\mu$ environ, comme des microbilles de latex. Ces microbilles peuvent fixer des protéines comme cela a été indiqué par SINGER, PLOTZ, Am. J. Med., (1956), 21, 888.

Un des avantages de l'invention réside dans le fait que les enzymes utilisées comme marqueurs n'ont pas besoin d'être purifiées, ce qui manifestement simplifie le procédé et le rend moins coûteux.

Les anticorps utilisés pour préparer le produit coupleur intermédiaire selon l'invention sont avantageusement des anticorps monoclonaux préparés comme décrit dans « Nature (1975) 256, 495 par Köhler et Milstein.

Des anticorps polyclonaux anti-enzyme peuvent aussi être utilisés, et sont préparés selon des méthodes connues (J. L. GUESDON Handbook of Experimental Immunology, ed. D. M. WEIR, Blackwell Scientific Publication, 1973).

Les résultats obtenus lors de leur application aux dosages sont équivalents à ceux obtenus avec les anticorps monoclonaux, si l'état de pureté des anticorps polyclonaux est suffisant.

Le couplage des anticorps est réalisé par l'intermédiaire de molécules symétriques choisies parmi la benzoquinone et le glutaraldéhyde mais principalement selon le procédé dont le principe est décrit par S. AVRAMEAS dans Immunochemistry 6, 43 (1969), c'est-à-dire par action du glutaraldéhyde pour créer un pont fixé par liaison covalente aux deux anticorps. Il n'est pas nécessaire que les deux anticorps qui seront couplés proviennent d'animaux de même race, ce qui est un autre avantage de l'invention. Par contre, le produit résultant du couplage doit être soluble dans les solutions aqueuses couramment utilisées dans ce type de dosage biologique, et les conditions de la préparation sont choisies en conséquence. Selon un procédé de préparation préféré, dans un tampon phosphate à pH voisin de 7, on dissout une certaine quantité de l'anticorps dirigé contre la substance antigénique à doser, tel qu'un anti-IgE ou un anti-IgG, pour obtenir une solution contenant de 20 à 100 mg/100 ml. On ajoute ensuite de 1 à 10 fois le poids du premier anticorps, d'un anticorps anti-enzyme, puis une solution aqueuse à 1 % de glutaraldéhyde, de telle sorte que le mélange final contienne une concentration de glutaraldéhyde comprise entre 0,02 % à 0,5 % (concentration finale). La durée d'incubation du mélange à une température d'environ 37 °C est fonction des concentrations relatives des trois composés ; elle peut être d'une demi-heure à plus de cinq heures. La réaction de pontage est ensuite stoppée par addition de glycine (concentration finale 0,1 M).

Si nécessaire, le produit coupleur peut alors être isolé par des méthodes connues de l'homme du métier.

Le procédé selon l'invention permet de doser de faibles quantités de substances, puisqu'on obtient déjà un résultat fiable lorsque la concentration de la substance antigénique dans le milieu est d'environ 1 ng/ml si c'est un antigène protéique et 5 à 10 ng/ml si c'est un anticorps. Il est aussi plus précis que les procédés antérieurs utilisant une enzyme directement liée à un anticorps, le pouvoir amplificateur du système selon l'invention étant supérieur à celui des systèmes précédemment décrits.

L'enzyme utilisée, qui permet la révélation peut être d'origine bactérienne, animale ou végétale ; il suffit qu'il existe une réaction, pour la révéler, de mise en œuvre sûre et facile.

Ladite enzyme aura par exemple un substrat fluorigène ou chromogène ; ou peut être fixée sur des particules insolubles d'un diamètre compris entre 0,5 et 20 microns environ.

Un autre objet de l'invention est le coffret de réactifs qui permet la mise en œuvre du procédé selon l'invention et qui contient :

— un anticorps ou antigène de la substance à doser immobilisé sur une matrice insoluble

— un produit coupleur résultant du pontage covalent entre un antigène ou anticorps de la substance à doser et un anticorps anti-enzyme

— ladite enzyme

— un système révélateur de ladite enzyme

Les exemples ci-après, non limitatifs, illustrent l'invention.

Les matrices sont des puits ou godets, ménagés dans des microplaques du commerce constituées d'un produit sur lequel peut être immobilisé l'anticorps de la substance à doser ; un tel produit peut être du polystyrène, sur lequel la fixation de l'anticorps est réalisée par un procédé connu en soi, et par exemple par contact d'une solution tamponnée de l'anticorps durant quelques heures à 37 °C puis durant 1 journée environ à 4 °C. Les plaques avec microgodets en U ou en V sont commercialisées par exemple par GREINER en France.

La présente invention concerne également des matrices constituées d'un support magnétique ou non tel que le Magnogel[(R)] (Pharm-industrie) préparé selon le brevet FR-A-2 334 106, ou de polyacrylamide ou d'agarose ou tout autre substance permettant d'immobiliser un réactif biologique.

Les immunosérums de mouton anti- IgE humaines ou antiperoxydase (de radis noir), et les immunosérums de lapin anti β-D-galactosidase d'E. coli utilisés peuvent être obtenus en hyperimmunisant les animaux avec les protéines correspondantes mélangées à de l'adjuvant complet de Freund (produit vendu par les Laboratoires Difco-Détroit USA) comme décrit dans Immunochemistry *6*, 43-53 (1969).

Les anticorps polyclonaux dirigés contre les IgE humaines ou la péroxydase peuvent être isolés de ces immunosérums par chromatographie d'affinité avec des immunoadsorbants préparés avec de l'Ultrogel[(R)] (Marque de Pharm-Industrie) comme décrit dans J. Immunol. Meth. *11*, 129 (1976). Les anticorps polyclonaux dirigés contre la β-galactosidase peuvent être isolés de l'immunosérum par chromatographie sur DEAE cellulose comme décrit dans Proc. Soc. Exp. Med. Biol. *103*, 250 (1960).

Les anticorps monoclonaux anti-peroxydase et anti-β-galactosidase peuvent être préparés par exemple selon la technique décrite par Köhler et Milstein dans Nature *256* 495 (1975).

La mesure de l'activité enzymatique de la matrice ayant subi les différentes étapes du procédé, permet de déterminer la quantité d'enzyme fixée à la matrice par l'intermédiaire des différents produits (antigène ou anticorps immobilisé, substance à doses, conjugué hybride anticorps-anticorps), et peut être réalisée de façon classique, en mettant l'enzyme en présence d'une solution convenable de son substrat ; on en déduit la quantité de substance à doser fixée et donc celle de départ.

Par exemple, pour la β-galactosidase, on introduit dans le godet de la microplaque 0,2 ml d'une solution aqueuse de tampon phosphate 0,1 M à pH 7 (M étant mis pour mole/litre) contenant 2,67 mM d'o-nitrophénylgalactropyranoside, 0,1 M de β-mercaptoéthanol, 1 mM de sulfate de magnésium, 0,2 mM de sulfate de manganèse et 2 mM de Titriplex (produit vendu par la Société Merck, Darmstadt-RFA). La plaque est alors incubée à l'obscurité, à 37 °C ; cette incubation peut durer de 1 à 4 heures et on choisit arbitrairement une durée dans ces limites. En fin d'incubation, on inactive l'enzyme par addition de 50 µl de carbonate de sodium 2 M et mesure l'absorption de la lumière par la solution résultante à une longueur d'onde de 414 nm.

Pour la peroxydase, on introduit par exemple dans le godet, 0,2 ml d'une solution aqueuse de pH 4,8 contenant 4 mg/ml d'o-phénylènediamine et 0,06 % (en poids) d'eau oxygénée et tamponnée avec une solution de 0,05 M de citrate de sodium. Les plaques sont incubées 10 minutes à l'obscurité, à 37 °C. La réaction enzymatique est alors interrompue par addition dans le godet de 50 µl de HCl 3N. L'absorption

**0 122 833**

est lue à 492 nm.

Lorsqu'on fait réagir le mélange constitué du conjugué hybride anticorps-anticorps et de l'enzyme servant de révélateur choisie, avec la matrice modifiée c'est-à-dire sur laquelle a été fixée la substance à doser par l'intermédiaire de son antigène (ou de son anticorps), ce mélange est effectué par exemple dans une solution de tampon phosphate sans modification des quantités d'anticorps et d'enzyme utilisées par rapport à celles des réactions successives.

Exemple 1

Dosage des immunoglobulines humaines IgE dans le sérum humain

A — Préparation des réactifs

Dans cet exemple, on utilise comme produit intermédiaire, le résultat de la réaction de couplage par le glutaraldéhyde de l'anticorps anti-IgE humaines avec de l'anticorps anti-β-galactosidase de E. coli effectuée comme suit :

On introduit dans 0,9 ml de tampon phosphate 0,1 M, à pH 6,8, 0,5 mg d'anticorps de mouton anti-IgE humaines et 2 à 4 mg d'anticorps monoclonaux anti-β-galactosidase, ou 1 à 2 mg d'anticorps polyclonaux anti-β-galactosidase, puis sous agitation douce 0,1 ml d'une solution aqueuse à 1 g/100 ml de glutaraldéhyde. Le mélange réactionnel est laissé 2 heures à température ambiante. Une autre variante consiste à ajouter 0,1 ml de solution à 5 % de glutaraldéhyde et à ne laisser incuber qu'une demi-heure. Ensuite, 50 μl d'une solution aqueuse 2 M de glycine sont ajoutés. Le mélange réactionnel est alors laissé 2 heures à température ambiante avant centrifugation et dialyse une nuit à 4 °C contre du tampon phosphate (PBS) ; on le mélange ensuite avec un même volume de glycérol bidistillé. La solution peut être ainsi conservée à — 20 °C pendant plusieurs mois jusqu'à son emploi.

B — Dosage

Les godets d'une plaque de polystyrène sont recouverts d'anticorps de mouton anti-IgE humaines : on introduit dans chacun 0,1 ml d'une solution de carbonate de sodium 0,1 M (pH = 9,6) contenant 0,1 μg d'anticorps anti-IgE, la plaque est incubée 2 heures à 37 °C, puis 18 heures à 4 °C et lavée. Ensuite 0,1 ml du sérum à étudier, convenablement dilué, est introduit dans chaque godet ainsi recouvert et la plaque mise à incuber pendant 18 heures à 4 °C.

(Le sérum à étudier est dilué avant son dosage par addition d'une solution saline tamponnée de phosphate contenant 0,1 g/100 ml de TWEEN-20[(R)] (esters d'acides gras et sorbitol polyéthoxylés, fournisseur Merck) et 0,3 g/100 ml de gélatine de telle sorte que la concentration des IgE humaines à doser soit comprise entre environ 100 U.l/ml et 0,2 U.l/ml).

Après la fin de l'incubation, la plaque est lavée 3 fois avec une solution saline tamponnée de phosphate contenant 0,1 g/100 ml de TWEEN-20 puis on ajoute dans chaque godet une solution du produit de couplage des deux anticorps précédemment préparés, ladite solution étant diluée si nécessaire pour que la concentration en anti-IgE couplé, soit d'environ 5 μg/ml.

Après une incubation de deux heures, à 37 °C, la plaque est de nouveau lavée trois fois et 0,1 ml d'une solution contenant environ 50 μg/ml de l'enzyme choisie comme marqueur, c'est-à-dire la β-galactosidase, est ajouté dans chaque godet et la plaque est laissée incuber 2 heures. Après les lavages habituels, 0,2 ml d'une solution du substrat de la β-galactosidase, préparée comme décrit précédemment, sont ajoutés et la révélation est effectuée selon une méthode classique.

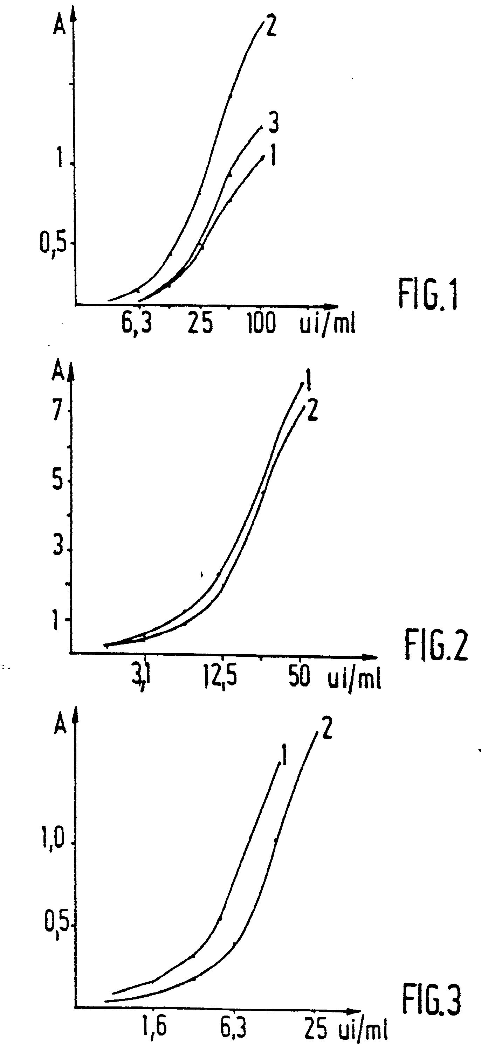

Dans la figure 1 est représentée l'absorption de la solution finale, lue à 414 nm en fonction de la concentration en IgE du sérum humain à étudier, exprimée en U.l/ml. Cette concentration a été déterminée préalablement à des fins de comparaison, par une méthode immunoenzymatique classique, en utilisant un sérum de référence normalisé, comme décrit dans J. Allergy Clin. Immunol. *61* 23 (1978). La relation entre l'absorption et la concentration en IgE est fonction du produit de couplage entre les deux anticorps. La courbe 1 correspond au dosage d'IgE en utilisant un conjugué hybride préparé en couplant 0,5 mg d'anticorps de mouton anti-IgE humaines avec 1 mg d'anticorps monoclonaux anti-β-galactosidase ; pour la courbe 2, on a utilisé 0,5 mg d'anticorps anti-IgE humaines avec 4 mg d'anticorps monoclonaux anti-β-galactosidase et pour la courbe 3, on a utilisé respectivement 0,5 mg et 8 mg.

Exemple 2

Autre dosage des immunoglobulines humaines IgE dans le sérum humain

Dans cet exemple, l'enzyme marqueur est la peroxydase extraite du radis noir, type I (indice de pureté : 0,6). Le produit intermédiaire résultant du couplage par le glutaraldéhyde de l'anticorps anti-IgE avec l'anticorps anti-peroxydase est préparé en suivant le mode opératoire décrit dans l'exemple 1, en faisant réagir 0,5 mg d'anticorps de mouton anti-IgE avec 2 mg d'anticorps anti-péroxydase.

La figure 2 donne la représentation graphique de l'absorption mesurée finalement en fonction de la concentration en IgE du sérum étudié, exprimée en unités internationales par ml. (U.I/ml). La courbe 1 a été obtenue avec des anticorps monoclonaux de souris, la courbe 2 avec des anticorps polyclonaux de mouton. En outre, on a appliqué cette méthode au dosage de différents sérums contenant des concentrations connues d'IgE et en utilisant des lots de péroxydase de puretés variables. Les résultats obtenus sont consignés dans le tableau 1. On constate que la concentration minimale d'IgE décelable par cette méthode est indépendante de l'état de pureté de l'enzyme : on dose 0,4 à 0,8 unités internationales d'IgE par ml, que la préparation de péroxydase utilisée soit très purifiée (indice de pureté : 3,0) ou brute (indice de pureté : 0,57).

## Exemple 3

Dosage simplifié des IgE dans le sérum humain

On utilise comme conjugué hybride, un produit préparé comme dans l'exemple 2, en solution dans du tampon phosphate. On mélange 0,1 ml de cette solution avec 0,1 ml de la solution de péroxydase (50 µg/ml) et introduit ce mélange dans les godets sur les parois desquels on a fixé préalablement l'anticorps anti-IgE puis le sérum à étudier, comme décrit dans l'exemple 1. Après une incubation de deux heures, la plaque est lavée et la révélation est conduite de manière classique.

Le dosage est plus rapide, car une étape et une incubation sont supprimées, cependant la méthode est un peu moins sensible que celle décrite dans l'exemple 2, comme le montre la figure 3. La courbe 1 a été tracée en utilisant le procédé décrit à l'exemple 2, la courbe 2 à l'exemple 3.

## Exemple 4

Dosage de l'anticorps anti-Echinococcus granulosus dans le sérum humain

On fixe dans les puits d'une plaque de microtitration en polystyrène, un antigène extrait de l'Echinococcus granulosus préparé à partir du liquide des kystes de moutons infectés par le parasite.

La fixation est réalisée en laissant dans les microgodets pendant 2 heures à 37 °C puis 18 heures à 4 °C, 0,1 ml de solution de tampon phosphate contenant 1 µg d'antigène. La plaque est ensuite lavée trois fois avec du tampon phosphate contenant 0,1 % de TWEEN[(R)]-20 puis 0,1 ml du sérum humain à étudier, dilué convenablement, sont introduits dans chaque godet. La dilution du sérum entre 1/200 et 1/204.800 environ, est réalisée avec du tampon phosphate contenant 0,1 % de TWEEN-20 et 0,3 % de gélatine.

La plaque, couverte, est incubée 2 heures à 37 °C, et lavée trois fois, puis on ajoute dans chaque puits 0,1 ml de la solution, à 5 µg/ml du produit de couplage des deux anticorps ; la plaque est alors maintenue 2 heures à 37 °C avant trois lavages successifs avec la même solution tamponnée que précédemment.

Le produit de couplage entre l'anticorps anti-IgG humaines et l'anticorps anti-β-galactosidase utilisé a été préparé avec le glutaraldéhyde en utilisant le procédé décrit dans l'exemple 1 : on a utilisé 0,5 mg d'anticorps anti-IgG humaines, 4 mg d'anticorps anti-enzyme et 0,1 ml d'une solution à 1 % de glutaraldéhyde pour un volume total de 1 ml.

La révélation de la β-galactosidase est effectuée comme décrit à l'exemple 1.

On a comparé les résultats obtenus par ce procédé à ceux obtenus en utilisant un dosage immunoenzymatique connu, mettant en œuvre un anticorps anti-IgG marqué par la phosphatase, celui-ci est préparé par réaction de 5 mg d'anticorps de lapin anti-IgG humaines avec 10 mg de phosphatase alcaline de E. coli en présence de glutaraldéhyde, comme décrit dans Immunochem. 6 43-53 (1969).

Au lieu d'ajouter le produit de couplage des deux anticorps selon l'invention dans les godets, on ajoute 0,1 ml d'une solution à 5 µg/ml de l'anticorps marqué par l'enzyme résultant de la réaction de couplage. On laisse la plaque 2 heures à 37 °C avant de la laver plusieurs fois ; on effectue alors l'étape de révélation en ajoutant 0,2 ml d'une solution 1 M de NaCl contenant 0,1 M de tampon Tris/HCl (ph = 8) et 1 mM de phosphate de p-nitrophénol ; après 16 heures à 37 °C, la réaction enzymatique est arrêtée par addition de 50 µl de $K_2HPO_4$ 3 M dans chaque puits et l'absorption lumineuse, due au p-nitrophénol, est mesurée à 405 nm.

Le tableau II donne les résultats de cette étude comparée. On constate que le procédé selon l'invention est beaucoup plus sensible.

(Voir Tableaux I et II pages 6)

# 0 122 833

Tableau I

Dosage des IgE humaines avec le conjugué hybride « anti-IgE/anti-péroxydase »

| IgE (c en UI /ml) | Péroxydase lot 1 | Péroxydase lot 2 | Péroxydase lot 3 | Péroxydase lot 4 |
|---|---|---|---|---|
| 100,0 | 0,972 | 0,907 | 0,869 | 0,620 |
| 50,0 | 0,776 | 0,790 | 0,710 | 0,549 |
| 25,0 | 0,620 | 0,533 | 0,489 | 0,371 |
| 12,5 | 0,372 | 0,318 | 0,247 | 0,224 |
| 6,3 | 0,207 | 0,174 | 0,130 | 0,115 |
| 3,1 | 0,094 | 0,092 | 0,053 | 0,040 |
| 1,6 | 0,052 | 0,05: | 0,027 | 0,022 |
| 0,8 | 0,023 | 0,02' | 0,010 | 0,008 |
| 0,4 | 0,009 | 0,010 | 0,000 | 0,000 |
| 0,2 | 0,000 | 0,000 | 0,000 | 0,000 |

Lot 1 : péroxydase dans du sulfate d'ammonium (indice de pureté : 3,0)

Lot 2 : péroxydase lyophilisée (indice de pureté : 3,0)

Lot 3 : péroxydase lyophilisée (indice de pureté : 1,7)

Lot 4 : péroxydase lyophilisée (indice de pureté : 0,57)

c = concentration.

Tableau II

Dosage comparatif des anticorps anti-Echinococcus granulosus dans le sérum d'un malade ou d'un témoin, selon la technique utilisant un conjugué hybride anti-IgG/anti-β-galactosidase (Ac-Gz) ou selon la technique classique de l'anticorps couplé avec la phosphatase alcaline (Ac/P).

| dilutions du sérum | Absorptions | | | |
|---|---|---|---|---|
| | Ac-P | | Ac-Gz | |
| | M | T | M | T |
| 1/400 | – | – | 0,919 | 0,082 |
| 1/800 | 1,979 | 0,265 | 0,755 | 0,059 |
| 1/1600 | 1,620 | 0,155 | 0,493 | 0,028 |
| 1/3200 | 1,281 | 0,094 | 0,339 | 0,037 |
| 1/6400 | 0,682 | 0,083 | 0,189 | 0,030 |
| 1/12800 | 1,341 | 0,052 | 0,103 | 0,023 |
| 1/25600 | 0,201 | 0,026 | 0,032 | 0,019 |
| 1/51200 | 0,105 | 0,002 | | |
| 1/102400 | 0,039 | 0,000 | | |

M = sérum de malade

T = sérum témoin

**Revendications** (pour les Etats contractants : BE, CH, DE, FR, GB, IT, LI, LU, NL, SE)

1. Procédé de dosage en phase hétérogène de substances antigéniques qui consiste :

a) à faire réagir l'échantillon contenant la substance à doser avec un anticorps (ou un antigène) immobilisé sur une matrice insoluble,

b) à mettre en contact la matrice ayant réagi selon a) avec un produit coupleur composé d'un anticorps (ou antigène) de la substance à doser et d'un anticorps anti-enzyme pontés par l'intermédiaire

6

de molécules symétriques choisies parmi la benzoquinone et le glutaraldéhyde, qui leur sont liées par covalence,

c) à faire réagir ensuite l'enzyme correspondant à l'anticorps composant le produit coupleur,

d) à doser l'enzyme fixée à la matrice modifiée au cours des étapes a) à c).

2. Procédé selon la revendication 1, caractérisé par le fait qu'on effectue simultanément les étapes b) et c).

3. Procédé selon l'une des revendications 1 et 2, caractérisé par le fait que la substance à doser est une immunoglobuline.

4. Procédé selon l'une des revendications 1 à 3, caractérisé par le fait que l'enzyme a un substrat chromogène ou fluorigène.

5. Procédé selon l'une des revendications 1 à 4, caractérisé par le fait que l'enzyme est choisie parmi la bêta-galactosidase de E. coli, la peroxydase et la phosphatase.

6. Procédé selon l'une des revendications 1 à 5, caractérisé par le fait que l'enzyme est fixée sur des micro-particules insolubles.

7. Ensemble de réactifs pour le dosage immunologique de substances antigéniques selon le procédé tel que décrit à l'une des revendications 1 à 6, caractérisé par le fait qu'il contient :

— un anticorps (ou antigène) de la substance à doser immobilisé par une matrice,

— un produit coupleur, composé d'un anticorps (ou antigène) de la substance à doser et d'un anticorps anti-enzyme, pontés par l'intermédiaire de molécules symétriques choisies parmi la benzoquinone et le glutaraldéhyde, qui leur sont liées par covalence,

— une enzyme,

— le système de révélation de l'enzyme.


**Revendications** (pour l'Etat contractant AT)

1. Procédé de dosage en phase hétérogène de substances antigéniques qui consiste :

a) à faire réagir l'échantillon contenant la substance à doser avec un anticorps (ou un antigène) immobilisé sur une matrice insoluble,

b) à mettre en contact la matrice ayant réagi selon a) avec un produit coupleur composé d'un anticorps (ou antigène) de la substance à doser et d'un anticorps anti-enzyme pontés par l'intermédiaire de molécules symétriques choisies parmi la benzoquinone et le glutaraldéhyde, qui leur sont liées par covalence,

c) à faire réagir ensuite l'enzyme correspondant à l'anticorps composant le produit coupleur,

d) à doser l'enzyme fixée à la matrice modifiée au cours des étapes a) à c).

2. Procédé selon la revendication 1, caractérisé par le fait qu'on effectue simultanément les étapes b) et c).

3. Procédé selon l'une des revendications 1 et 2, caractérisé par le fait que la substance à doser est une immunoglobuline.

4. Procédé selon l'une des revendications 1 à 3, caractérisé par le fait que l'enzyme a un substrat chromogène ou fluorigène.

5. Procédé selon l'une des revendications 1 à 4, caractérisé par le fait que l'enzyme est choisie parmi la bêta-galactosidase de E. coli, la peroxydase et la phosphatase.

6. Procédé selon l'une des revendications 1 à 5, caractérisé par le fait que l'enzyme est fixée sur des microparticules insolubles.


**Claims** (for the Contracting States : BE, CH, DE, FR, GB, IT, LI, LU, NL, SE)

1. Heterogeneous phase assay method for antigenic substances, which consists in :

a) reacting the sample containing the substance to be assayed with an antibody (or an antigen) immobilized on an insoluble matrix,

b) bringing the matrix, which has reacted according to a), into contact with a coupling product composed of an antibody (or antigen) to the substance to be assayed and an anti-enzyme antibody bridged via symmetrical molecules chosen from benzoquinone and glutaraldehyde, which molecules are bound to them covalently,

c) then reacting the enzyme corresponding to the antibody which composes the coupling product, and

d) assaying the enzyme bound to the matrix modified during the stages a) to c).

2. Method according to Claim 1, characterized in that the stages b) and c) are performed simultaneously.

3. Method according to one of Claims 1 and 2, characterized in that the substance to be assayed is an immunoglobulin.

4. Method according to one of Claims 1 to 3, characterized in that the enzyme has a chromogenic or fluorogenic substrate.

5. Method according to one of Claims 1 to 4, characterized in that the enzyme is chosen from beta-galactosidase of E. coli, peroxidase and phosphatase.

6. Method according to one of Claims 1 to 5, characterized in that the enzyme is bound to insoluble micro-particles.

7. Kit of reagents for the immunoassay of antigenic substances according to the method as described in one of Claims 1 to 6, characterized in that it contains :
— an antibody (or antigen) to the substance to be assayed,
— the antibody (or antigen) being immobilized by a matrix, a coupling product composed of an antibody (or antigen) to the substance to be assayed and an anti-enzyme antibody bridged *via* symmetrical molecules chosen from benzoquinone and glutaraldehyde, which molecules are bound to them covalently,
— an enzyme, and
— the system for visualizing the enzyme.


**Claims** (for the Contracting State AT)

1. Heterogeneous phase assay method for antigenic substances, which consists in :
a) reacting the sample containing the substance to be assayed with an antibody (or an antigen) immobilized on an insoluble matrix,
b) bringing the matrix, which has reacted according to a), into contact with a coupling product composed of an antibody (or antigen) to the substance to be assayed and an anti-enzyme antibody bridged *via* symmetrical molecules chosen from benzoquinone and glutaraldehyde, which molecules are bound to them covalently,
c) then reacting the enzyme corresponding to the antibody which composes the coupling product, and
d) assaying the enzyme bound to the matrix modified during the stages a) to c).

2. Method according to Claim 1, characterized in that the stages b) and c) are performed simultaneously.

3. Method according to one of Claims 1 and 2, characterized in that the substance to be assayed is an immunoglobulin.

4. Method according to one of Claims 1 to 3, characterized in that the enzyme has a chromogenic or fluorogenic substrate.

5. Method according to one of Claims 1 to 4, characterized in that the enzyme is chosen from beta-galactosidase of E. coli, peroxidase and phosphatase.

6. Method according to one of Claims 1 to 5, characterized in that the enzyme is bound to insoluble micro-particles.


**Patentansprüche** (für die Vertragsstaaten : BE, CH, DE, FR, GB, IT, LI, LU, NL, SE)

1. Verfahren zum Dosieren von antigenen Substanzen in heterogener Phase, bestehend aus :
a) Einwirkenlassen der Probe, die die zu dosierende Substanz enthält, auf einen auf einer unlöslichen Matrix immobilisierten Antikörper (oder ein Antigen),
b) Inkontaktbringen der gemäß a) umgesetzen Matrix mit einem Kupplungsprodukt, zusammengesetzt aus einem Antikörper (oder Antigen) der zu dosierenden Substanz und aus einem Antienzym-Antikörper, überbrückt durch symmetrische Zwischenmoleküle, ausgewählt aus Benzochinon und Glutaraldehyd, die daran kovalent gebunden sind,
c) anschließendem Einwirkenlassen des Enzyms das zu dem Antikörper entspricht, der das Kupplungsprodukt bildet,
d) Dosieren des auf der im Ablauf der Schritte a) bis c) modifizierten Matrix fixierten Enzyms.

2. Verfahren nach Anspruch 1, dadurch gekennzeichnet, daß die Schritte b) und c) gleichzeitig durchgeführt werden.

3. Verfahren nach einem der Ansprüche 1 und 2, dadurch gekennzeichnet, daß die zu dosierende Substanz ein Immunoglobulin ist.

4. Verfahren nach einem der Ansprüche 1 bis 3, dadurch gekennzeichnet, daß das Enzym ein chromogenes oder fluorogenes Substrat hat.

5. Verfahren nach einem der Ansprüche 1 bis 4, dadurch gekennzeichnet, daß das Enzym aus beta-Galactosidase von E. coli, Peroxidase und Phosphatase ausgewählt ist.

6. Verfahren nach einem der Ansprüche 1 bis 5, dadurch gekennzeichnet, daß das Enzym auf unlöslichen Mikroteilchen fixiert ist.

7. Test-kit der Reagenzien zur immunologischen Dosierung antigener Substanzen nach dem in einem der Ansprüche 1 bis 6 beschriebenen Verfahren, dadurch gekennzeichnet, daß es enthält :
— einen Antikörper (oder ein Antigen) der zu dosierenden, durch eine Matrix immobilisierten Substanz,

— ein Kupplungsprodukt, zusammengesetzt aus einem Antikörper (oder Antigen) der zu dosierenden Substanz und aus einem Antikörper-Antienzym, überbrückt durch symmetrische Zwischenmoleküle, ausgewählt aus Benzochinon und Glutaraldehyd, die daran kovalent gebunden sind,

— ein Enzym,

— das System um das Enzym erkennen zu lassen.

**Patentansprüche** (für den Vertragsstaat AT)

1. Verfahren zum Dosieren von antigenen Substanzen in heterogener Phase, bestehend aus :

a) Einwirkenlassen der Probe, die die zu dosierende Substanz enthält, auf einen auf einer unlöslichen Matrix immobilisierten Antikörper (oder ein Antigen),

b) Inkontaktbringen der gemäß a) umgesetzen Matrix mit einem Kupplungsprodukt, zusammengesetzt aus einem Antikörper (oder Antigen) der zu dosierenden Substanz und aus einem Antienzym-Antikörper, überbrückt durch symmetrische Zwischenmoleküle, ausgewählt aus Benzochinon und Glutaraldehyd, die daran kovalent gebunden sind,

c) anschließendem Einwirkenlassen des entsprechenden Enzyms das zu dem Antikörper entspricht der das Kupplungsprodukt bildet,

d) Dosieren des auf der im Ablauf der Schritte a) bis c) modifizierten Matrix fixierten Enzyms.

2. Verfahren nach Anspruch 1, dadurch gekennzeichnet, daß die Schritte b) und c) gleichzeitig durchgeführt werden.

3. Verfahren nach einem der Ansprüche 1 und 2, dadurch gekennzeichnet, daß die zu dosierende Substanz ein Immunoglobulin ist.

4. Verfahren nach einem der Ansprüche 1 bis 3, dadurch gekennzeichnet, daß das Enzym ein chromogenes oder fluorogenes Substrat hat.

5. Verfahren nach einem der Ansprüche 1 bis 4, dadurch gekennzeichnet, daß das Enzym aus beta-Galactosidase von E. coli, Peroxidase und Phosphatase ausgewählt ist.

6. Verfahren nach einem der Ansprüche 1 bis 5, dadurch gekennzeichnet, daß das Enzym auf unlöslichen Mikroteilchen fixiert ist.

FIG.1

FIG.2

FIG.3